(19) Europäisches Patentamt
European Patent Office
Office européen des brevets

(11) EP 2 749 317 B1

(12) **EUROPEAN PATENT SPECIFICATION**

(45) Date of publication and mention
of the grant of the patent:
**23.03.2016 Bulletin 2016/12**

(51) Int Cl.:
***A61N 5/06*** (2006.01)

(21) Application number: **13199648.0**

(22) Date of filing: **27.12.2013**

(54) **Wearable apparatus for nail treatments**

Tragbare Vorrichtung zur Nagelbehandlung

Dispositif portable pour traitements des ongles

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **27.12.2012 GB 201223438**

(43) Date of publication of application:
**02.07.2014 Bulletin 2014/27**

(73) Proprietors:
• **Medical Quant Ltd.**
**44425 Kfar Saba (IL)**
• **M.B. Mazor Consulting and Services Ltd**
**Hod Hasharon (IL)**
• **Presburger, Gadi**
**Tel Aviv (IL)**
• **Tenne, Asaf**
**Yahud (IL)**
• **Thein, Amos**
**Yahud (IL)**

(72) Inventors:
• **Povolosky, Moshe**
**deceased (IL)**
• **Gotlieb, Gal Elchanan**
**Ra'anana 43398 (IL)**

(74) Representative: **Lecomte & Partners**
**P.O. Box 1623**
**1016 Luxembourg (LU)**

(56) References cited:
**WO-A1-2013/005156     US-A1- 2008 058 905
US-A1- 2009 143 842**

**Description**

**FIELD OF INVENTION**

This invention pertains to apparatuses for treating fungal nail infections, more specifically this invention provides an apparatus for radiation therapy treatment of fungal nail infections.

**BACKGROUND**

Fungal infections are commonly treated in various methods, compositions and devices. Current treatments of fungal infections include systemic antifungal medications, topical treatment and laser treatment. Different medical treatments have been known including antifungal oral medications, nail creams, ointments and lacquers. The problem with the medical treatment and methods is that many of them have proved to be ineffective or require a long period of time for curing the fungal nail infection and further produced considerable side effects. Photodynamic therapy (PDT) has been proposed as an enticing alternative of eliminating fungi in toenail infections because it provides an interesting mechanism of action comprising a photosensitizer which is activated by a suitable light irradiation wavelength. The use of laser radiation has been an ever-growing method of treating Onychomycosis. Recent studies indicate laser treatment to be effective in 85% of the eligible treated toenails. Laser wavelengths are able to penetrate thickened nail plate and eliminate fungus residing on nail bed while lacking teratogenic effects associated with ultraviolet light, thus making the laser safe for use. Furthermore, research states that ease of delivery and lack of need to monitor blood chemistry are attractive attributes to using laser treatments, regardless of the degree of severity. Such therapies are further suggested in the following publications:

US patent No. 2007167999 discloses a multi-spectra photon therapy apparatus comprising an array of LEDs having at least two sets of LEDs configured according to a predetermined pattern, with a first set of LEDs emitting light in the about red wavelength and at least a second set of LEDs emitting light in the about infrared wavelength; a frequency generator for modulating a signal to the LED array; and a controller for selecting the frequency applied to the signal and the duration during which the signal is modulated at the selected frequency; a protocol database including a plurality of predetermined sequences of frequencies and durations of time specific to treat or ameliorate predetermined diseases or conditions.

US patent No. 2007104664 discloses an apparatus that provides heat and/or ultraviolet light to fingernails or toenails that are afflicted by Onychomycosis. The apparatus may be used in connection with systemic and/or topical antifungal agents to treat Onychomycosis. US patent No. 2008058905 discloses an apparatus that utilizes one or more blue light emitting diodes (LED) to irradiate fungus residing under and around the nail is applied externally to a toe or finger in order to kill the fungus and restore normal nail growth. The light therapy is applied in this manner periodically at scheduled times. US patent No. 2008076958 discloses apparatus for treating hard biological tissue such as fungal infections with electromagnetic energy having a frequency of at least 0.5 MHZ (megahertz) and less than 10 GHZ (gigahertz) such as HF, RF or microwave energy. US patent No. 2009234270 discloses a light delivery apparatus for use in photodynamic treatment of Onychomycosis comprising (i) a housing adapted to cover a nail beneath which a locus is situated; (ii) a light source; (iii) an energy source in power communication to the light source; and a controller that controls amount of light to be applied to the locus by the light source a method and apparatus for treating fungal infections. The procedure further includes: applying a composition which includes a photosensitizer, an effective amount of antifungal agent, and a pharmaceutically acceptable delivery system to a locus and irradiating the locus with a light source at a wavelength absorbed by the photosensitizer so as to destroy microbes at the locus.

International patent application published WO 2013/005156 A1 (date of publication: 10-01-2013) is a prior right document in the sense of Art. 54(3) of the European Patent Convention. The disclosure of this application relates to device and method of treating fungal nail infection by using a first type of light which is a blue light of a wavelength of between 450 nm and 500 nm and a second type of light which is a red light generated by a red laser diode with a wavelength of between 600 nm and 950 nm.

All the above mentioned treatments have limitations among which are low efficacy with high recurrence rates, side effects, and high costs. Therefore, a cost-effective, self-treating home medical device, safe, easy to use and with the curing effectiveness of laser treatment is necessary.

It therefore remains a long felt and unmet need to provide novel means for an effective treatment for fungal nail infections in more effective manner and in parallel reducing treatment and curing time period.

## SUMMARY OF THE INVENTION

The invention is defined in the appended set of claims. Methods of treating fungal infections mentioned hereinafter do not form part of the present invention.

It is one object of the present invention to provide a portable apparatus useful treating fungal nail infections, comprising: (a) a toe clip or a finger clip, and an irradiator having at least one light emitting diode (LED) source and at least one pulsed laser light source ; said irradiator being mounted in the clip for irradiating the area of interest, (b)a controller comprising machine readable medium for controlling the light sources and, (c)a power source, wherein the controller is provided with predetermined protocols for instructing the light sources of the irradiator to emit light LED and pulsed laser light at predetermined wavelengths, frequencies and treatment intensity; further wherein projected beams of the light and pulsed laser light are configured to at least partially overlap on the area of interest such that it substantially covers all of the infected area.

It is one object of the present invention to provide a portable finger clip apparatus useful for treating fungal nail infections, comprising: (a)an irradiator having at least one light emitting diode (LED) source and at least one pulsed laser light source mounted therein for irradiating the area of interest, (b)a controller comprising machine readable medium for controlling said LED and pulsed laser light sources and, (c)a power source, wherein the controller is provided with predetermined protocols for instructing said at least one LED sourcesto emit light in a predetermined wavelength of between about 390 nm to about 500 nm and for instructing said at least one pulsed laser light in a predetermined wavelength of between about 600 nm to about 950 nm; further wherein sait at least one LED source and said at least one pulsed laser light source are arranged in a geometric array such that the projected beams of said at least one LED light source and said at least one pulsed laser light source are configured to at least partially overlap said area of interest, thereby providing maximal efficacy.

It is one object of the present invention to provide in an irradiator for irradiating an area having fungal infections, the irradiator comprising at least one light emitting diodes (LED) source and at least one pulsed laser light source mounted therein, a controller comprising a machine readable medium for controlling the light sources and, a power source, wherein the controller is provided with predetermined protocols for instructing the light sources of the irradiator to emit LED light and pulsed laser light at predetermined wavelengths, frequencies and treatment intensity; further wherein projected beams of the light and pulsed laser light are configured to at least partially overlap on the area of interest such that it substantially covers all of the infected area.

It is one object of the present invention to provide a portable apparatus useful for treating fungal nail infections, comprising: (a)a housing for housing light sources for irradiating an area of interest on the nail; the housing comprises: (i)at least one light emitting diode (LED) source and, (ii)at least one pulsed laser light source,
(b)a controller comprising a machine readable medium for controlling the light sources and, (c) a power source, wherein the controller is provided with predetermined protocols for instructing the light sources of the irradiator to emit LED light and pulsed laser light at predetermined wavelengths, frequencies and treatment intensity; further wherein projected beams of the light and pulsed laser light are configured to at least partially overlap on the area of interest such that it substantially covers all of the infected area.

It is one object of the present invention to provide a portable apparatus for treating fungal nail infections, comprising: (a) an irradiator housed within the apparatus; the irradiator is configured to irradiate the area of interest with at least two light emitting diodes (LED) source and at least one pulsed laser light source,(b)a controller comprising a machine readable medium for controlling the light sources and, (c)a power source,
wherein the LED source and the pulsed laser light source are arranged in a geometric array such that the projected beams of the light sources are configured to overlap the area of interest; the geometric array provides optimal light distribution, such that the irradiator emits light in wavelength frequencies at a predetermined treatment intensity such that it substantially covers all of the infected area.

It is another object of the present invention to disclose the apparatus detailed above, wherein the application of the a predetermined protocol provides a treatment not exceeding pain level between 0 to 2 on the Wong Baker faces scale.

It is another object of the present invention to disclose the detailed above apparatus, wherein the geometric array has a configuration selected from the group consisting of: polygon, square, triangle, hexagon, sphere, hemisphere, bow-tie, cylinder, circle, ellipse, rectangles, T-shape, or any polygon arranged as a two-dimensional array.

It is another object of the present invention to disclose the detailed above apparatus, wherein the geometric array has a three-dimensional (3D) array configuration.

It is another object of the present invention to disclose the detailed above apparatus, wherein the configuration of the geometric array further comprises at least one light source positioned in any pattern within the interior of the configuration for distributing light.

It is another object of the present invention to disclose the detailed above apparatus, wherein the geometric array of the light sources for providing an optimal overlapped light beam on the area of interest is adjusted according to variables selected from the group consisting of: the position of the light sources, the intensity of the light sources, the distance between each light source, the optimal frequency for treating the area of interest and any combination thereof.

It is another object of the present invention to disclose the detailed above apparatus, wherein the controller is configured to operate the laser at a pulse repetitive rate (PRR) mode, whilst the LED sources are taken as a quasi continuous operation.

It is another object of the invention to disclose the detailed above apparatus, wherein the geometry arrangement of the light sources of said laser and at least the one LED are within an acceptance angle adjusted to about 6.9 mm distance between the light sources.

It is another object of the present invention to disclose the detailed above apparatus, wherein the light sources produce an extended source having average acceptance angle of about 35 mR.

It is another object of the present invention to disclose the detailed above apparatus, wherein the apparatus has a structure for fitting over a patient's finger nail/ toe nail such that the apparatus is discretely wearable.

It is another object of the present invention to disclose the detailed above apparatus, wherein the apparatus has a structure of a toe or finger clip that includes a lower portion and an upper portion that are opposed to one another and each pivotally attached to a spring portion to keep the clip closed around a toe or finger, the clip incorporates at least one light LEDs and at least one pulsed laser light mounted in the clip in order to emit light toward a nail when the clip is in a closed configuration around a toe or finger.

It is another object of the present invention to disclose the detailed above apparatus, wherein the apparatus further comprises a separator configured to at least partially separate the irradiator and/or other portions of the apparatus from the area of interest. It is another object of the present invention to disclose the detailed above apparatus, wherein the fungal infections are selected from the group consisting of: distal lateral subungual Onychomycosis (DLSO), white superficial Onychomycosis (WSO), proximal subungual Onychomycosis (PSO), endonyx Onychomycosis (EO), candidal Onychomycosis and any related disorder.

It is another object of the present invention to disclose the detailed above apparatus, wherein the blue light source is of a wavelength of between about 390 nanometer (nm) and about 500 nm.

It is another object of the present invention to disclose the detailed above apparatus, wherein the LED source is with a beam divergence of about 9x25 degree.

It is another object of the present invention to disclose the detailed above apparatus, wherein the pulsed laser light source is of a wavelength of between about 600nm and about 950nm.

It is another object of the present invention to disclose the detailed above apparatus, wherein the pulsed laser light source is with a beam divergence of about 120 degree.

It is another object of the present invention to disclose the detailed above apparatus, wherein the red laser light source combined with LED light source confers a synergistic effect on the area of interest when compared to the therapeutic effect of the LED source.

It is another object of the present invention to disclose the detailed above apparatus, wherein the pulsed laser light source combined with LED light source confers a synergistic effect on the area of interest when compared to the therapeutic effect of the red laser light source.

It is another object of the present invention to disclose the detailed above apparatus, wherein the irradiator is used for irradiating the red light according to a frequency scheme including a first sequence of frequencies increasing continuously from a first frequency to a second, greater, frequency, and a second sequence of frequencies decreasing continuously from the second frequency to the first frequency.

It is another object of the present invention to disclose the detailed above apparatus, wherein the first frequency includes a frequency of about 100 Hertz (Hz), and the second frequency includes a frequency of about 3000 Hz.

It is another object of the present invention to disclose the detailed above apparatus, wherein the irradiator is used for irradiating the light during a first irradiation period, and to irradiate the pulsed laser light during a second irradiation period.

It is another object of the present invention to disclose the detailed above apparatus, wherein the first and second irradiation periods at least partially overlap.

It is another object of the present invention to disclose the detailed above apparatus, wherein the apparatus further comprises a rechargeable battery as the power source.

It is another object of the present invention to disclose the detailed above apparatus, wherein the light sources emit pulsed or continuous laser radiation of wavelength in the near infrared region of the electromagnetic spectrum.

It is another object of the present invention to disclose the detailed above apparatus, wherein the light sources are administrated in pulsed manner.

It is another object of the present invention to disclose the detailed above apparatus, wherein the geometric array provides optimal distribution and intensity of light per Watt.

It is another object of the present invention to disclose the detailed above apparatus, wherein the geometric array provides optimal distribution and intensity of light so as to provide maximal efficacy.

In a preferred embodiment, the apparatus has a structure of a toe or finger clip that includes a lower portion and an upper portion that are opposed to one another, each portion being pivotally attached to a spring portion to keep the clip closed around a toe or finger, wherein the clip incorporates at least one light LED and at least one pulsed laser light mounted in the clip in order to emit light toward a nail when the clip is in a closed configuration around a toe or finger.

## BRIEF DESCRIPTION OF THE DRAWINGS

In order to understand the invention and to see how it may be implemented in practice, a few preferred embodiments will now be described, by way of non-limiting example only, with reference to be accompanying drawings, in which:

Fig. 1 is a schematic illustration of the apparatus of the present invention;

Figs. 2A and 2B are schematic illustrations of the apparatus positions and configurations for treating fungal nail infections;

Figs. 3A and 3B are schematic illustrations of the apparatus positions and configurations for treating fungal nail infections;

Figs. 4A, 4B and 4C are schematic illustrations of an isometric view of the apparatus of the present invention for treating fungal nail infections;

Fig. 5 presents a frequency graph as a function of time of the present invention;

Fig. 6 illustrates a graph of the treatment of the apparatus of the present invention according to a predetermined protocol;

Fig. 7 is a schematic flow-chart illustration of a method of treating fungal nail infections of the present invention;

Fig. 8A is schematic illustration of the geometric configuration array of the apparatus of the present invention;

Fig. 8B is schematic illustration of the power effect of the geometry structure of the light sources within the apparatus of the present invention; and

Fig 9 is a perspective view of a subject's finger nail during treatment using the apparatus of the present invention;

## DETAILED DESCRIPTION OF THE INVENTION

In the following description, various aspects of the invention will be described. For the purposes of explanation, specific details are set forth in order to provide a thorough understanding of the invention. It will be apparent to one skilled in the art that there are other embodiments of the invention that differ in details without affecting the essential nature thereof. Therefore the invention is not limited by that which is illustrated in the figures and described in the specification, but only as indicated in the accompanying claims, with the proper scope determined only by the broadest interpretation of the claims.

The term *"fungal infection"* specifically applies hereinafter to Onychomycosis also known as "Dermatophytic Onychomycosis," "Ringworm of the nail," and "Tinea unguium "Distal subungual onychomycosis" , "White superficial onychomycosis" (WSO), "Proximal subungual onychomycosis", "Candidal onychomycosis" or any other disorder or disease related to fungal infection of toenails or fingernails.

The present invention provides a portable and wearable apparatus for non-invasive treatment of fungal nail infections which functions as an autonomous unit. The apparatus of the present invention is configured to be a pocket device having a small weight and size so that it can be easily carried. The apparatus further provides anti-bacterial and anti-microbial effects. The apparatus comprises: (a) an irradiator, which includes at least one blue light LED source and at least one IR laser red light source, (b) a controller comprising a machine readable medium or central processing unit (CPU) for controlling and activating the irradiator, and (c) a power source such as a rechargeable battery.

The present invention provides the combination of Low Level Laser Therapy (LLLT) and visible blue light in one apparatus, the energy of which penetrates through the infected nail, targeting fungus spores residing underneath. This combination provides a synergic therapeutic, anti microbial and anti inflammatory effect which traverses the infected nail, and functionally inhibits or destroys or arrests growth of the fungal spores.

The apparatus of the present invention achieves an efficient solution in easing and curing symptoms associated with fungal infection of the nail, both for toenails and for fingernails. The apparatus comprises a low level laser which together with blue light radiation produces a therapeutic effect, curing fungal nail infection both in the feets as well as in the hands. These two different energy modalities, combined together, will produce a symbiotic effect of eliminating Onychomycosis.

The apparatus of the present invention further provides a treatment for nail fungal infection of both finger and toe nails. The apparatus may further be adapted for the aesthetic and cosmetic use. The apparatus further fulfills the need for safe, non-invasive and user friendly treatment.

The apparatus of the present invention provides a Low level laser Treatment (LLLT). Low-level laser therapy is known as a medical and veterinary treatment that uses low-level lasers or light-emitting diodes to alter cellular function. Low level laser therapy is used in the treatment of a broad range of conditions. LLLT improves wound healing, reduces edema, and relieves pain of various etiologies, including successful application to wound and surgical sites to reduce inflammation and pain. The apparatus of the present invention provides LLLT which further adapted to reduce pain according to a pain scale. The pain scale rates subject's level of pain therefore, measures a patient's pain intensity or other features. Different types of pain scales may further be adapted such as verbal, numerical or visual pain scales. The pain scale which may be used is Wong Baker Faces Pain Scale having the following scale: Face 0 doesn't hurt at all, Face 2 hurts just a little bit, Face 4 hurts a little more, Face 6 hurts even more, Face 8 hurts a whole lot and Face 10 hurts as much as you can imagine. The apparatus of the present invention is with a pain Wong Baker Faces Pain Scale level between face 0 to face 2.

LLLT is further adapted in the treatment protocol to repair of injured muscles and tendons. LLLT utilizes low level laser energy, wherein the treatment has a dose rate that causes no immediate detectable temperature rise of the treated tissue and no macroscopically visible changes in tissue structure. Consequently, the treated and surrounding tissue is not heated or damaged, and the patient feels no sensation during treatment. LLLT may further effectively photodestroy a targeted biological element under suitable treatment conditions.

The effects of LLLT is limited to a specified set of wavelengths of laser, and administering LLLT below the dose range does not appear to be effective.

Without wishing to be bound by theory ,LLLT may further reduce pain related to inflammation by lowering, in a dose-dependent manner, levels of prostaglandin E2, prostaglandin-endoperoxide synthase 2, interleukin 1-beta, tumor necrosis factor-alpha, the cellular influx of neutrophil granulocytes, oxidative stress, edema, and bleeding. The appropriate dose appears to be between 0.3 and 19 joules per square centimetre. Another mechanism may be related to stimulation of mitochondrion to increase the production of adenosine triphosphate resulting in an increase in reactive oxygen species, which influences redox signalling, affecting intracellular homeostasis or the proliferation of cells. The final enzyme in the production of ATP by the mitochondria, cytochrome c oxidase, does appear to accept energy from laser-level lights, making it a possible candidate for mediating the properties of laser therapy. The effects of LLLT is limited to a specified set of wavelengths of laser, and furthermore it is required to determine the ideal wavelengths, durations of treatment, dose and location of treatment. Administering LLLT below the dose range does not appear to be effective. The typical laser average power is in the range of 1-500 mW, some high-peak-power, short-pulse-width devices are in the range of about 1-100 W with typical pulse-widths of 200 ns. Therefore the typical average beam irradiance is then 10 mW/cm$^2$-5 W/cm$^2$. The typical wavelength is in the range of about 600-1000 nm (red to near infrared).

The apparatus is activated according to a predetermined protocol combining Blue LED and IR laser light at selected frequencies according to selected sequences. The controller is provided for instructing the light sources of the irradiator to emit blue LED light and IR laser light at predetermined wavelengths, frequencies and treatment intensity. Furthermore, projected beams of the blue LED source and IR laser light source are configured to overlap the area of interest such that it substantially covers all of the infected area

The irradiator is configured to irradiate the area of interest with light of at least two light sources having two different wavelengths .The light sources combined together in a specific intensity eliminate and/or destroy the nail fungal infection and in parallel provides skin and/or nail therapy of the area of interest. The light sources of the apparatus are configured to emit adequate light from at least two light sources into a patient's nail in a manner that is adjusted to cure the fungal nail infection of a subject. In another embodiment of the present invention, the combination of the red laser light source with the blue LED source confers a synergistic effect on the area of interest when compared to the therapeutic effect of only a blue LED source. The combination of the red laser light source with the blue LED source confers a synergistic effect on the area of interest when compared to the therapeutic effect of only a red laser light source.

The present invention further provides a portable and in parallel wearable apparatus for non-invasive treatment of fungal nail infections which comprises: (a) an irradiator which includes at least two blue light LED and at least one IR laser, (b) a controller for activating and controlling the irradiator and, (c) a power source such as a rechargeable battery. The apparatus of the present invention is a portable device and does not need any exterior power source.

The blue LED source and the IR laser light source are arranged in an optimized geometric array such that the projected beams of the light sources are configured to overlap the area of interest. The geometric array provides optimal light distribution, such that the irradiator emits light in wavelength frequencies at a predetermined treatment intensity which substantially covers all of the infected area. The geometric array further provides optimal light distribution therapy.

The geometric array structure is determined according to a predetermined angle and distance between the light sources which are selected for higher and improved emission efficacy,

There are several possible uniform light patterns such as regular polygons, using squares, triangles or hexagons. The geometric array structure may be spheres, hemispheres, cylinders, or squares arranged as a two-dimensional array. A similar structure that is also in accordance with the present invention is a two-dimensional array shaped as circles, ellipses, squares, rectangles, triangles, or bow ties. The geometric array may further include a three-dimensional (3D) array configuration.

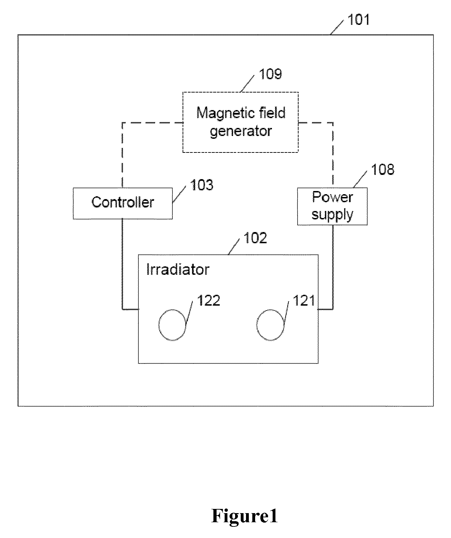

Reference is now made to FIG. 1, which shows a portable apparatus for treating fungal nail infections 101, comprising; (a) an irradiator 102 for housing light sources for irradiating an area of interest on a nail. The housing comprises at least one blue light emitting diode (LED) source 122 and at least one IR laser light source 121, (b) a controller 103, and (c) a power source

The apparatus of the present invention is configured as a pocket device having a small weight and size such that it can be easily carried.

In another embodiment of the present invention, apparatus 101 may include a magnetic field generator 109 configured to generate a magnetic field around the area of interest. The magnetic field provides a symbiotic effect for treating the fungal nail infection, in which a penetration of the red and blue lights may be at a deeper depth of the treated area. In other embodiments, the apparatus 101 may not utilize the magnetic field.

In another embodiment of the present invention, the irradiator is adapted for emitting light of wavelength frequencies at a predetermined treatment intensity according to a predetermined protocol by combining Blue LED and IR laser light sources according to selected sequences. The apparatus is further adjusted to treat the area of interest such that it covers completely all of the infected area.

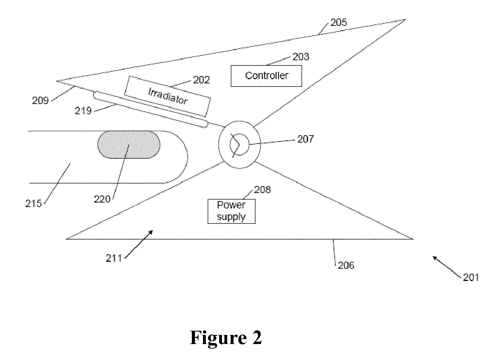

In another embodiment of the present invention, the apparatus includes a power supply unit 108 configured to supply power required for the operation of apparatus 101 to activate the light sources 121 and/or 122, to the controller 103 comprising a CPU and/or to any other suitable element of apparatus 101. Power supply unit 108 may include any suitable portable power supply unit such as a battery, a rechargeable battery, and the like so as to create a discretely autonomous unit. In other embodiments, apparatus 101 may receive electric power from an outer supply source via a suitable electric connector. The apparatus of the present invention is configured as a pocket device having a small weight and size so that it can be easily carried. Reference is now made to **FIG. 2** which presents the positioning angle and the wearable configuration of the apparatus upon the area of interest. The apparatus 205 has a clip structure which is wearable upon a toe or finger. In another embodiment of the present invention, the clip apparatus designed to fit over a toe or a finger in a manner similar to clips worn on the finger for use in pulse oximetry devices.

In another embodiment of the present invention the structure of the apparatus may further include circular designs, ring designs, clip designs, C-shaped designs, sock designs, thimble designs, cup-like designs that wrap the whole finger or glove finger designs.

In another embodiment of the present invention the apparatus is easy to carry such that it may further be adapted as a key chain, ring or the like.

The apparatus structure is configured to increase user comfort by enabling the user to perform various daily activities without any substantial interference. The apparatus is formed of any suitable material configured to perform the functionality of a clip, any suitable plastic material and/or rubber material and the like. One or more elements of apparatus 201 may be housed within clip 211 such as an irradiator 202. The inner surface of the apparatus 201may be made from any suitable material configured to enable a convenient and comfortable use of apparatus 201 such as a soft rubber gasket and/or the like.

The apparatus includes gripping elements comprising a lower portion 206 and an upper portion 205 that are opposed to one another and each is pivotally attached to a spring portion 207 to keep the clip closed around a toe or finger 220. The clip incorporates at least one blue light LED and at least one IR laser light source mounted in the clip in order to emit light toward a nail when the clip is in a closed configuration around a toe or finger. Therefore it is adequately attached to a toe or a finger.

The laser beam is encapsulated within the apparatus in a manner which prevents any projection or contact with subject's eyes such that no eye damage is occurred when light is transmitted. Therefore the apparatus is safe for the home user.

In another embodiment of the present invention, the apparatus may include an attaching element configured to position the irradiator at an affixed position suitable to irradiate the area of interest. The apparatus may further include an attaching element such as an hinge spring which is configured to maintain gripping elements 205 and 206 in a closed position, thus tight around subject finger 215. The user may simultaneously press the gripping elements 205 and 206 to pivotally separate gripping elements 205 and 206 to enable placing and affixing a subject finger between gripping elements 205 and 206.

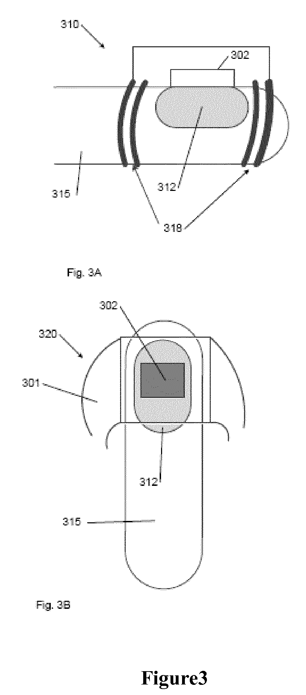

Reference is now made to **FIG. 3A-3B**, which illustrates a side view of the apparatus structure and position upon a finger or toe nail 312. The apparatus comprising the irradiator is positioned at a suitable and desired position for irradiating the area of interest 302.

The apparatus further provides a non-invasive treatment of the nail fungal infection without any need to use any traditional medical treatment, such as medicines, chemical substances, medical tests, visit at a clinic and follow up visits at the clinic and/or the like. The apparatus is intended for the end user, and does not require any sort of specialist consulting or specialist treatment. Furthermore if a medication is needed the apparatus may be adjusted to provide treatment comprising the medication. The apparatus of the present invention is an over the counter product (OTC), furthermore it does not require any medical prescription.

In another embodiment of the present invention, the apparatus may be configured to be reused on the same finger or toe and/or on a different finger or toe of the same user or of a different user.

In another embodiment of the present invention, the apparatus may further include a separator configured to at least partially separate the irradiator and/or other portions of the apparatus from the area of interest. The separator may include a replaceable separator, which may be replaced between treatments of the same nail and/or between treatments of different nails. Accordingly, the separator may reduce the risk of, or prevent, contamination by the nail fungi of the area of interest. The separator may further comprise silicon pads which may be interchangeable or suitable for a single use.

The apparatus of the present invention comprises at least two sources of lights. The first light source is a blue LED source, with a wavelength of between about 390 nanometer (nm) to about 500nm, more preferable about 470nm. The apparatus may further utilizes one or more blue light emitting diodes (LED) to irradiate fungus residing under and around the nail and is applied externally to a toe or finger in order to treat the fungus and restore normal nail growth. Light therapy may be applied in this manner periodically at scheduled times according to the predetermined protocol. Without wishing to be bound by theory, the fungal nail infection is alleviated by the effect of blue light on porphyrins without causing damage to the skin in the treated area. Further more, irradiating the treated area with the blue light may damage the DNA structure of the fungal nail infection, which prevents the fungal nail infection from being able to replicate. Furthermore, blue light radiation creates free radicals, which may split bonds between molecules of the fungal nail infection, resulting in destroying the molecules, at least partially. Irradiating the area of interest with the blue light source has a sanitizing and/or sterilizing effect on the nail bed and the tissue which surrounds the treated nail. Furthermore, irradiating the area of interest with the blue light source can ease and cure one or more symptoms associated with the fungal nail infections and ease lateral skin infections caused by fungal nail infection side effects. The intensity of the blue light is not limited and it can be adjusted to patients of various ages and/or having various health conditions, including regular usage of medicine containing harmful cross-effects with conventional oral or other types of medications. The intensity can be adjusted between 3W -9W. Furthermore, blue light has an antibacterial effect, and does not cause deoxyribonucleic acid damage or early photo-ageing to healthy skin tissue. The biological effects of blue light on normal skin and,or nail are transient melanogenesis and inexplicable vacuolization without resulting apoptosis. The use of a blue light LED source in dermatological practice is considered to be safe.

In another embodiment of the present invention, in order to increase treatment effectiveness a second light source is further directed at the site of infection. The second type of light is of an infra red (IR) light which includes at least one IR laser light diode with a wavelength of a visible and near IR region of the electromagnetic spectrum. The wavelength is between about 600to about 950nm, preferably about 905nm. The red light has a relatively low power level, a high peak power of about 25w. Irradiation, by the red light having a low power level of about 25W, does not cause any side effects such as side effects on the skin surrounding the treated area, e.g., burn marks, blisters, scars, redness of skin, irritation and/or the like, which may be related to using laser of a relatively high bandwidth, such as a wavelength above 1000nm and/or an ultraviolet light. Therefore, utilizing the red light which has a relatively low power level is safer for use. Furthermore, irradiating the area of interest with the red light does not involve any pain during and/or after the treatment.

The infra red (IR) light source provides a low level laser therapy (LLLT) which can be used to treat fungal nail infections.

Without wishing to be bound by theory, The LLLT works by penetrating thickened nail and enhancing one of the most fundamental molecular mechanisms in the human body - cellular respiration and the production of ATP. As more ATP is produced, energy consuming biological work such as movement, protein synthesis and active transport will become enhanced. The result is that inflamed and damaged tissue surrounding infected nail will heal faster. Moreover, increased cellular activity due to treatment by the apparatus of the present invention will enable faster growth of new and healthy nails, thus substantially shortening time needed for treatment results to show.

Without wishing to be bound by theory, current thinking is that the red light changes the biochemical behavior of ATP molecules and may relatively increase the production of ATP. Therefore irradiating the area of interest with the red light increases the production of ATP molecules thus, enhances biological work, which requires chemical energy, such as, movement, protein synthesis, active transport and, or the like. It is known that the ATP molecules are the "fuel" that drives protein production, cell proliferation and further stores the chemical energy and release chemical energy to the biochemical processes occurring in the cell. Therefore the infra red (IR) light source encourages the growth and production of healthy tissue.

Furthermore, irradiating the treated area with the red light reduces the time period, which is required for growing new and healthy nails. For example, irradiating the treated area with the red light may reduce a treatment time from about 9 months to less then about 3 months.

In another embodiment of the present invention the projected beams of the blue light and IR light provide an effective amount of low-level laser energy to inhibit fungal cell growth. The light sources emit a low-level laser energy which has a therapeutically effective amount per square centimeter.

The apparatus of the present invention further includes a controller comprising a CPU or a machine readable medium configured to control the functionality and/or the operation of the apparatus and/or irradiator, as described above. The controller controls the irradiator such that it will generate a red light according to a predetermined protocol. The controller controls the irradiator function such that it will generate a blue light according to a predetermined protocol. The controller further controls the irradiator to generate a blue light source combined with a red light source according to a suitable predetermined protocol.

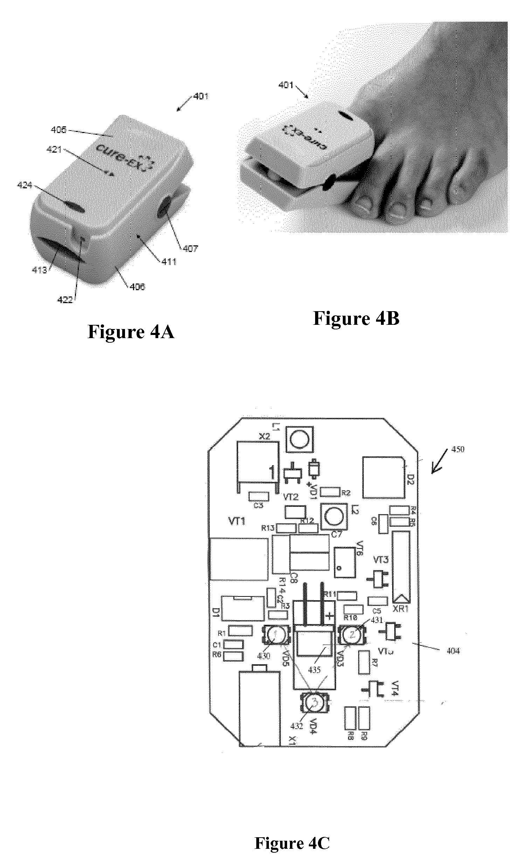

Reference is now made to **Fig. 4A-C**, which illustrates an isometric view of an apparatus 401 for treating fungal nail infections. Fig. 4B further illustrates utilization of apparatus 401 on a toenail of a user. Fig. 4C illustrates an electronic circuit 450 of apparatus 401. The gripping element 406 may be curved in a shape of an arch 413, corresponding to a shape of an outer surface of the area of interest.

In another embodiment of the present invention, the apparatus 401 may include a user interface configured to enable the user to operate apparatus 401 and/or to indicate to the user a mode of operation of apparatus 401. The user interface of apparatus 401 may include an on/off power button 424 configured to operate apparatus 401, to switch apparatus 401 between a first mode ("on") and a second mode ("toff"). Power button 424 may include any suitable button, switch or the like. The apparatus 401 may be switched to the second mode automatically without any intervention of a user of apparatus 401. For example, apparatus 401 may switch apparatus 401 to the "off" mode after a predefined time period after turning on apparatus 401, e.g., 7 minutes.

In another embodiment of the present invention, the user interface of apparatus 401 may include at least one indicator 421. The indicator indicates to the user of the mode of operation of the apparatus and/or the mode of operation of one or more light sources of the apparatus. The apparatus may include two separate indicators, a first indicator, which may indicate an operational mode of the blue light source, and a second indicator, which may indicate an operational mode of the red light source. Indicator may include any suitable indicator such as, a two-color LED, and the like. Indicator 421 may be configured to indicate the mode of operation of apparatus 401 and/or a mode of operation of one or more light sources of apparatus 401. The apparatus 401 may include two separate indicators, a first indicator, which may indicate an operational mode of the blue light source, and a second indicator, which may indicate an operational mode of the red light source. Indicator 421 may include any suitable indicator, a two-color LED, and the like.

In another embodiment of the present invention, the apparatus 401 may include a battery charger connector 422, configured to be connected to an external battery charger in order to charge the power supply unit. Charging the power supply unit may be implemented using any suitable method, via wireless charging and/or a charging pad.

As shown in Fig. 4C, the electronic circuit 450 may include a laser diode 435 configured to generate the red light. Laser diode 435 may perform the functionality of red light source.

In some demonstrative embodiments, electronic circuit 450 may include three blue LEDs 430, 431 and 432, configured to generate the blue light such as blue LEDs 430, 431 and 432 may perform the functionality of blue light source.

In another exemplary embodiment of the present invention, the electronic circuit 450 may include a main board 404 configured to electrically connect between elements of apparatus 401. Electronic circuit 450 may include suitable electronic elements such as resistors, capacitors, diodes and/or transistors, as listed in Table 1 as follows:

**Table 1**

| Part number | Name | Qty | Remarks |
|---|---|---|---|
| | Capacitors | | |
| C1 | CHIP-0603-X7R-4,7 mkF-10V | 1 | |
| C2 | CHIP-0603-X7R-0,1 mkF-50V | 1 | |
| C3 | CHIP-0603-X5R-10 mkF-6,3V | 1 | |
| C4 | CHIP tantalum-C case-220mkF-6,3V | 1 | |
| C5 | CHIP-0603-X7R-4,7 mkF-6,3V | 1 | |

(continued)

| Part number | Name | Qty | Remarks |
|---|---|---|---|
| C6 | CHIP-0603-X7R-0,1 mkF-50V | 1 | |
| C7 | CHIP-1206-Y5V-4,7 mkF-50V | 1 | |
| C8 | CHIP-1206-0,1 mkF-50V (CL31B104KBCNNNC) | 1 | SAMSUNG |
| | Integrated Circuit | | |
| D1 | ADP2291ARMZ (MSOP-8) | 1 | Analog Apparatuss,Inc |
| D2 | PIC16F1827-I/ML (QFN-28) | 1 | Microchip Technology,Inc |
| G1 | Li-Polymer Battery LP753048 | 1 | EEMB Co.,Ltd |
| L1 | CHIP inductors SH3018 100YL | 1 | ABC Taiwan Electronics |
| L2 | CHIP inductors SH3018 470YL | 1 | ABC Taiwan Electronics |
| | Resistors | | |
| R1 | CHIP-0805-0,22 Om +-1% | 1 | |
| R2 | CHIP-0603-130 kOm +-5% | 1 | |
| R3 | CHIP-0603-200 kOm +-5% | 1 | |
| R4 | CHIP-0603-20 kOm +-1 % | 1 | |
| R5 | CHIP-0603-15 kOm+-1% | 1 | |
| R6 | CHIP-0603-470 Om +-5% | 1 | |
| R7-R9 | CHIP-0805-75 Om+-5% | 3 | |
| R10 | CHIP-0603-2,2 Om+-5% | 1 | |
| R11 | CHIP-0603-470 Om +-5% | 1 | |
| R12 | CHIP-0603-10 kOm +-5% | 1 | |
| R13 | CHIP-0603-130 kOm +-5% | 1 | |
| R14 | CHIP-1206-620 kOm +-5% | 1 | |
| S1 | Tactics button TSQGA-T-1.5 H=1,5mm | 1 | TOP-UP CORP |
| VD1 | Diode PMEG2010EJ. (SOD323F) | 1 | |
| VD2 | BI-COLOR Red/Green LED KPB-3025SURKCGKC | 1 | Kingbright |
| VD3-VD5 | BLU LED ET-3528B-A11W | 4 | Edison Opto |
| VD6 | Diode MBR0540T1 (SOD-123) | 1 | |
| VD7 | Laser Diode SPL PL90-3 | 1 | OSRAM |
| VT1 | Transistor PBSS5540Z (SOT-223) | 1 | Termal area |
| VT2 | Transistor IRLML6346TR (SOT-23) | 1 | |
| VT3-VT5 | Transistor IRLML6401TR (SOT-23) | 3 | |
| VT6 | Transistor IRF7752TR (TSSOP-8) | 1 | |
| X1 | Plug TPJ338S-SMT | 1 | TOP-UP CORP |
| X2 | Plug DS1066-2MRW6 (2-Pins) | 1 | Connfly Co., Ltd |
| XR1 | Plug DS1025-01-5P6BV2 (5-pins) (2mm) | 1 | Connfly Co.,Ltd absent |

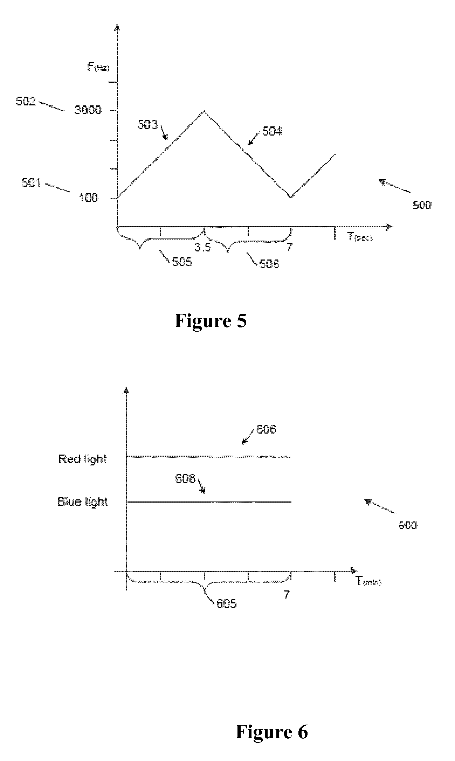

Reference is now made to **Fig. 5**, which presents graph of a treatment according to a predetermined protocol of frequencies 500. The predetermined protocol 500 is implemented by the apparatus of the present invention. The controller controls the irradiator to generate a red light according to a predetermined protocol configured to irradiate the area of interest throughout a predefined range of depths. The depth of the area of interest affected by the red light depends on the frequency of the red light source. The red light at a first frequency affects an area at a first depth, and red light at a second frequency, greater than the first frequency, affects an area at a second depth, less than the first depth.

As shown in Fig. 5, the predetermined protocol 500 may include a first sub-sequence 503 of frequencies, increasing continuously, from a first frequency 501, e.g., 100 Hertz (Hz), to a second frequency 502, e.g., 3000Hz, during a predefined time period 505, e.g., 3.5 seconds. Furthermore, as shown in Fig. 5, sub-sequence 503 may be followed by a second sub-sequence 504 of frequencies, e.g., decreasing continuously, from second frequency 502, e.g., 3000Hz, to first frequency 501, e.g., 100 Hz, during a predefined time period 506, e.g., 3.5 seconds.

In another embodiment of the present invention, the predetermined protocol 500 can be repeated throughout at least a portion of an irradiation period for irradiating the red light. The controller indicates the red light source to generate light according to the predetermined protocol 500, which may be repeated, for example, throughout a red-light irradiation period.

The predetermined protocol, may further define one or more first irradiation periods for irradiating the red light and one or more second irradiation periods for irradiating the blue light. The controller may further control red light source and blue light source to irradiate in parallel substantially, simultaneously, for a predefined period of time, such as about seven minutes, or any other predefined period.

In another embodiment of the present invention, the controller can control the irradiation of the red light source for a first predefined time period, and further controls the irradiation of blue light source for a second predefined period, which may be different from, e.g., longer than, the first time period or vice versa.

In another embodiment of the present invention, the first and second irradiation periods may at least partially overlap.

Reference is now made to **Fig. 6**, which illustrates a graph of the treatment of the apparatus of the present invention according to a predetermined protocol 600. The apparatus comprising a controller controls the irradiator to generate the blue and/or red light according to predetermined protocol 600. The predetermined protocol 600 may include a first irradiation period 608 for irradiating the red light and a second irradiation period 606 for irradiating the blue light. Both of the periods, 606 and 608, may be aligned to begin at substantially the same time, such that the red and the blue lights may be irradiated, substantially simultaneously, during a time period 605, such as about seven minutes.

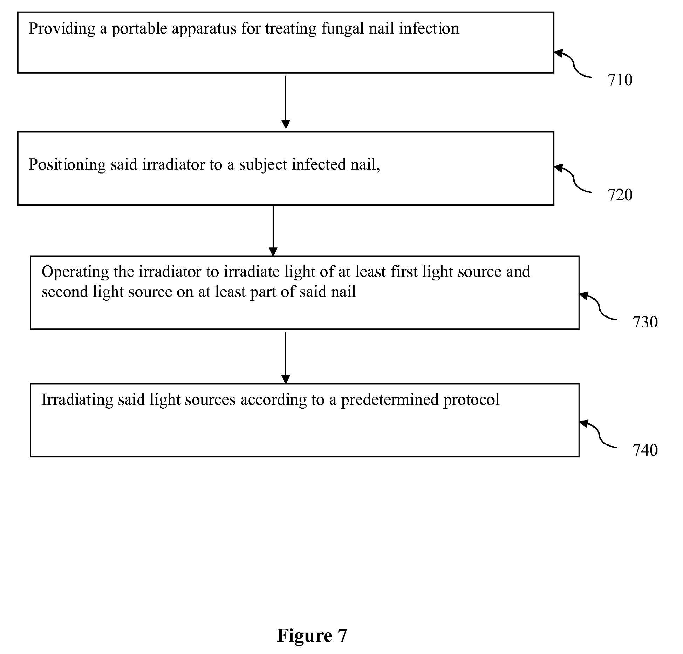

Reference is now made to **Fig 7**, which presents a method for irradiating an infected fungal nail, comprising the steps of: (a) providing a portable apparatus for treating fungal nail infections 710, comprising: (i) an irradiator housed within the apparatus; the irradiator is configured to irradiate the area of interest with at least one source of light such as blue light emitting diodes (LED) source and at least one red source of light such as IR laser light source, (ii) a controller comprising a machine readable medium, and (iii)a power source. (b) positioning the irradiator to a subject infected nail 720, (c)operating the irradiator to irradiate light of at least one blue light source and at least one red light source on at least a portion of the subject infected nail 730, (d) irradiating the light sources according to a predetermined protocol 740 including a first sequence of frequencies increasing linearly and continuously from a first frequency to a second, greater, frequency, and a second sequence of frequencies decreasing linearly and continuously from the second frequency to the first frequency.

The apparatus of the present invention is configured as a pocket device having a small weight and size so that it can be easily carried.

The blue LED source and the IR laser light source are arranged in a geometric array such that the projected beams of the light sources are configured to overlap the area of interest. The geometric array provides optimal light distribution, such that the irradiator emits light in wavelength frequencies at a predetermined treatment intensity such that it substantially covers all of the infected area without causing any damage to healthy tissue.

In another embodiment of the invention the method as described above, wherein the first source of light comprises a blue light of a wavelength of between about 390nm and about 500nm.

In another embodiment of the invention the method as described above, wherein the blue light comprises light of a wavelength of about 470nm.

In another embodiment of the invention the method as described above, wherein the red light source comprises a red light of a wavelength of between about 600nm and about 950nm.

In another embodiment of the invention the method as described above, wherein the red light comprises light of a wavelength of about 905nm.

In another embodiment of the invention the method as described above, wherein the first frequency includes a frequency of about 100 Hertz (Hz), and the second frequency includes a frequency of about 3000Hz.

In another embodiment of the invention the method as described above, further comprising irradiating the blue light source during a first irradiation period, and irradiating the red light source of light during a second irradiation period.

In another embodiment of the invention the method as described above, wherein the first and second irradiation periods at least partially overlap.

In another embodiment of the invention the method as described above, wherein the geometric array provides optimal distribution and intensity of light per Watt.

In another embodiment of the invention the method as described above, wherein the geometric array provides optimal distribution and intensity of light so as to provide maximal efficacy.

In another embodiment of the present invention the method as described above, wherein the apparatus has a structure of a toe or finger clip that includes a lower portion and an upper portion that are opposed to one another and each pivotally attached to a spring portion to keep the clip closed around a toe or finger. The clip incorporates at least one blue light LED and at least one IR laser light mounted in the clip in order to emit light toward a nail when the clip is in a closed configuration around a toe or finger. Therefore it is adequately attached to a toe or a finger.

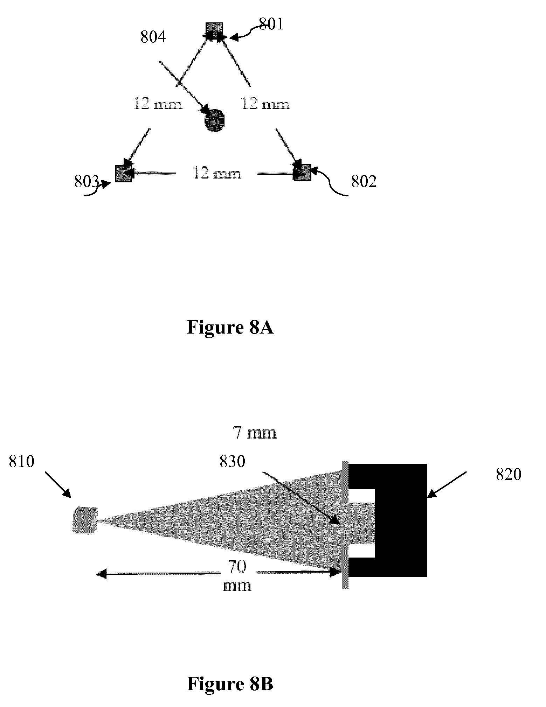

Reference is now made to **Fig 8A**, which presents the light sources configuration housed within the irradiator of the apparatus of the present invention. The irradiator contains one infra-red laser source 804 and 3 visible LEDs 801-803 arranged at the geometric structure of a triangle pattern 800. The irradiator may further include any suitable combination of one or more light sources. As shown in Fig. 8A the irradiator includes three LED sources 801,802,803 arranged at the geometric structure in a respective edge of a triangle whilst each LED is in an equal distance away from each other. Furthermore the triangle array comprises at least one red light source 804 located at the center of the triangle configuration 800, forming a triangular-pyramidal array structure. In order to eliminate and/or destroy the nail fungal infection and in parallel to provide skin therapy of the area of interest, the desired distance between each led light is of about 12mm . In order to define the optimized array of several light sources, an arrangement of light sources is selected which provides an optimal overlapped light beam on the treatment surface. The variables are: the position of the light sources, the intensity of the light source, the distance between each light source relative to the optimal geometric arrangement, the Frequency and wavelength adjusted to treat the fungal infection of the area of interest.

The optimal radiation pattern of a single light source may be compared to a respective required radiation pattern by calculating a desired distance of the light source from an optical axis of the lighting device.

Other possible uniform light patterns may be adapted such as regular

polygons, using squares, triangles or hexagons. The geometric array structure may further be of spheres, hemispheres, bow-ties, cylinders, or squares arranged as a two-dimensional (2D) array. The geometric array may further be a 3D configuration. A similar structure that is also in accordance with the present invention is a two-dimensional array shaped as., circles, ellipses, squares, rectangles, triangles, T-shape or bow ties. The array of light sources is designed to increase the efficiency of the intensity of light treatment upon the area of interest. The geometric arrangement of the light sources is specifically configured in a manner for minimizing the heat generated by the apparatus. The geometric arrangement further provides uniform radiation to the area of interest, whilst the apparatus configuration further targets uniform light beams to the infected area

The geometric arrangement is based upon the inverse-square law which states that the intensity of a specified physical quantity is inversely proportional to the square of the distance from the source of that physical quantity. In equation form:

$$\text{Intensity} \ \propto \ \frac{1}{\text{distance}^2}$$

This means that the intensity or irradiance of the emitted light or other linear waves radiating from a point source (energy per unit of area perpendicular to the source) is inversely proportional to the square of the distance from the source such that an object twice as far away, receives only one-quarter the energy (in the same time period). More generally, the irradiance, *i.e.*, the intensity (or power per unit area in the direction of propagation), of a spherical wave front varies inversely with the square of the distance from the source (assuming there are no losses caused by absorption or scattering).

In another embodiment of the present invention, the irradiator may comprise any other suitable combination of light sources, for example, two red light sources and four blue light sources, and the like. The blue and red light sources may be arranged in any suitable arrangement, e.g., in the form of a rectangle, a circle, and the like.

In another embodiment of the present invention, the light therapy may be administrated in a pulse therapy manner.

In another embodiment of the present invention at least one pulse parameter is selected from the group consisting of amplitude, pulse duration, a pulse shape, a duty cycle parameter, a pulse sequence parameter, a pulse rise-time, and a pulse frequency.

Light administration of the apparatus of the present invention is pulsed, and may be controlled by a controller such as microprocessor. Pulsing of the different light source may treat the affected nail whilst reducing the overall red and blue light exposure to the patient.

**Table 2** presents the laser beams specification

**Table 2**

| Source | λ | Output Power | Pulse energy | Pulse width | PRR | Beam Dimension | Beam Divergence |
|--------|---|--------------|--------------|-------------|-----|----------------|-----------------|
| Laser | 905 nm | 6 mW | 3 μl | 100 ns | 2 KHz * | 200x2 μm | 9x25 deg. |
| LED | 470 nm | 100 mW | Duty cycle 50% ** | | | ----- | 120° |

The laser is operated at Pulse Repetitive Rate (PRR) mode, where the LEDs are taken as a quasi continuous operation. The pulse repetition rate, also known as *pulse repetition frequency* of a regular train of pulses, is the number of emitted pulses per second, or the inverse temporal pulse spacing. If a pulse train is regular and the pulses are mutually coherent, the optical spectrum of the pulse train is a frequency comb, where the spacing of the lines is determined by the pulse repetition rate.

On the other hand, quasi-continuous-wave (quasi-cw) operation of a laser means that its pump source is switched on only for certain time intervals, which are short enough to reduce thermal effects significantly, but still long enough that the laser process is close to its steady state, i.e. the laser is optically in the state of continuous-wave operation. Therefore, quasi-cw operation allows the operation with higher output peak powers at the expense of a lower average power.

For repetitive pulses the accessible emission limit (AEL) class 1 may be calculated for three cases -Single pulse, Repetitive Pulse and Average power, where the most restrictive case determines the actual AEL. For LEDs the AEL Class 1 is determined by long exposure (average power).

**Table 3** presents the calculation formula for the laser and LED light sources.

**Table 3**

| Source | Single Pulse (**S.P.**) | Repetitive Pulses | Average Power |
|--------|------------------------|-------------------|--------------|
| Laser | $2 \cdot 10^{-7} C_4$ J | $S.P \cdot C_5$ | $3.9 \cdot 10^{-4} C_4$ W |
| LED | ------------ | ----------- | $3.9 \cdot 10^{-3} C_3$ & $3.9 \cdot 10^{-4}$ W |

Where, $C_4$ equal 2.57 for 905 nm $C_5$ equal 0.084 for 2 KHz PRR of a point source $C_3$ (the photochemical coefficient) is 2.5 for 470 nm

Substituting the correction factors values in the formulas of Table 3, we get the calculation results presented in **Table 4** below.

**Table 4**

| Source | Single Pulse (**S.P.**) | Repetitive Pulses | Average Power |
|--------|------------------------|-------------------|--------------|
| Laser | $5.14 \cdot 10^{-7}$ | $4.3 \cdot 10^{-8}$ J/Pulse | $10^{-3}$ W, $5 \cdot 10^{-7}$ J/Pulse |
| LED | | | $3.9 \cdot 10^{-4}$ W * |

The relevant sources that should be taken into account are those within 100 mR angle of acceptance. For a 100 mm distance, 100 mR defines 10 mm length. Using the arrangement geometry of the light sources, as presented in Fig. 8, shows that only the laser and 1 LED could be included within the relevant acceptance angle (6.93 mm distance between them). But those sources (laser + 1 LED) produce an extended source having average acceptance angle(α) of 35.25 mR, thus non point area, resulted in $C_6$ value of 23.5 (α/1.5). As a result the AEL of the two sources is multiplied by 23.5, where the total power increases much lower. The bottom line is that the two sources are less hazardous than each one of them. Therefore, the classification of a single source determines all the light sources of the apparatus.

Reference is now made to **Fig. 8B**, which presents the power effect of the geometry structure of the light sources within the apparatus of the present invention. The measurement was taken for the worst case scenario.

The apparatus was tested as follows: a laser radiation detector 820 was placed at 70 mm distance from the light source (LED or laser) whilst a single light source was operated in its normal current and the other 3 were disconnected. The detector 820 was placed with the 7 mm aperture 830 in front of it as close as to the apparatus ( worst case), where the device was opened to its largest opening. The apparatus was operated (laser and 3 LEDs (810)) and the results of the measurements are as presented in table 5.

**Table 5** below presents the expected power through 7 mm aperture and the measured one, for light sources classification, and the measurement results.

**Table 5**

| Classification | AEL Class 1 | AEL Class 3R | AEL Class 3B | Power Through 7 mm aperture | |
|---|---|---|---|---|---|
| | | | | Theory | Measurement |
| Laser | 0.086 mW | 0.43 mW | 500 mW | 1.5 mW | 0.205 mW |
| Single LED | 0.39 mW | 1.96 mW | 500 mW | 0.125 mW | 0.25 mW |
| Product | 5.8 mW | 29 mW | 500 mW | ------ | 0.105 mW |

As table 5 shows, there are differences between the theory and the power measurements. However, for both theory and measurements the possible exposure level (0.105 mW) is much lower than the AEL class 1 (5.8 mW), whilst the apparatus is assigned Class 1 laser product. Moreover, the light sources are totally enclosed into the protective housing in a way that direct exposure to the light sources is not possible.

The laser light source is encapsulated within the clip configuration of the apparatus of the present invention, which further provides a safer and focused light irradiation. The apparatus is safe to use without any irradiation leakage.

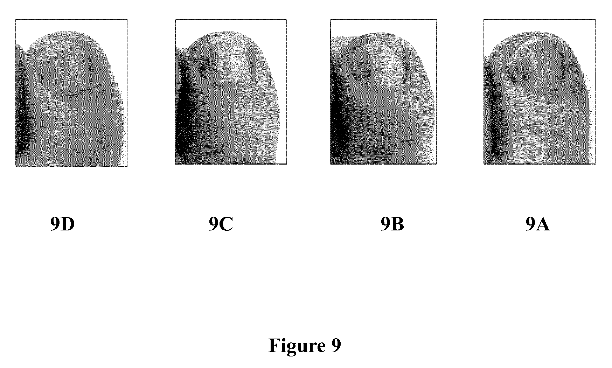

Reference is now made to **Fig. 9** which presents a case study, using the present invention, over time with several results of a subject's toe nail as the trial progressed. The study is intended to evaluate the efficacy of a treatment for the condition it is intended to treat; possible side effects are monitored.

**Figs. 9A-9D** demonstrate a case study of treating fungal nail infection with the apparatus of the present invention. The case study was conducted by the apparatus of treating fungal nail infections. As demonstrated in the case study described below, a nail infected by fungal nail infections, which was treated by the apparatus, showed an improvement of the nail health and esthetic appearance after a relatively short period of time, compared to common treatments of fungal nail infections.

**Fig. 9A** presents the condition of the subject's toe nail having a fungal nail infection, before starting a treatment.

**Fig. 9B** presents the condition of the subject's toe nail 5 weeks after starting treatment.

**Fig. 9C** presents the condition of the subject's toe nail 8 weeks after starting treatment.

**Fig. 9D** presents the condition of the subject's toe nail 10 weeks after starting treatment which shows an improvement of the nail health and esthetic appearance after a relatively short period of time.

**Claims**

1. A portable finger clip or toe clip apparatus (101) for treating fungal nail infections, comprising:

    a. a finger clip or toe clip and an irradiator (102) having at least one LED source (122) and at least one pulsed laser light source (121) ; said irradiator (102) being mounted in the clip for irradiating an area of interest;
    b. a controller (103) comprising a machine readable medium for controlling said at least one LED and pulsed laser light sources (122, 121) ;and
    c. a power source; wherein said controller (103) is provided with predetermined protocols (500) for instructing said at least one LED source (122) to emit first light at a predetermined wavelength of between 390 nm to 500 nm and for instructing said at least one pulsed laser light source (121) to emit second light at a predetermined wavelength of between 600 nm to 950 nm;

    further wherein said at least one LED source (122) and said at least one pulsed laser light source (121) are arranged in a geometric array such that the projected beams of said at least one LED source (122) and said at least one pulsed laser light source (121) are configured to at least partially overlap said area of interest, thereby providing maximal efficacy.

2. The apparatus (101) according to claim 1, wherein at least one of the following holds true:

    a. said geometric array has a configuration selected from the group consisting of: polygon, square, triangle, hexagon, sphere, hemisphere, cylinder, circle, ellipse, rectangles, T-shape, bow tie or any polygon arranged as a two-dimensional array; and

b. said geometric array has a three-dimensional (3D) or two-dimensional array configuration.

3. The apparatus (101) according to claim 1, wherein the distance between said at least one LED and pulsed laser light sources (121, 122) is 6.9 mm.

4. The apparatus (101) according to claim 1, wherein the at least one LED source (122) is configured to emit light at a wavelength of 470 nm.

5. The apparatus (101) according to claim 1, wherein said at least one pulsed laser light source (122) is configured to emit light at a wavelength of 905 nm.

6. The apparatus (101) according to claim 1, wherein said controller (103) is configured to operate said at least one pulsed laser light source (121) in a pulse repetitive rate mode, whilst said at least one LED light source is driven in a quasi-continuous mode.

7. The apparatus (101) according to claim 1, wherein said geometric array of light sources produces an extended light source having an average acceptance angle of 35 mR.

8. The apparatus (101) according to claim 1, wherein the toe or finger clip includes a lower portion and an upper portion opposed to one another, wherein each portion is pivotally attached to a spring portion to keep the clip closed around a toe or finger, wherein the clip is configured to emit said first and second light toward a nail when the clip is in a closed configuration around the toe or finger.

9. The apparatus (101) according to claim 1, comprising a separator configured to at least partially separate said irradiator and/or other portions of the apparatus from said area of interest.

10. The apparatus (101) according to claim 1, wherein said at least one LED source (122) is configured to provide a beam divergence of 9x25 degree.

11. The apparatus (101) according to claim 1, wherein said at least one pulsed laser light source (121) is configured to provide a beam divergence of 120 degree.

12. The apparatus (101) according to claim 1, wherein at least one of the following holds true:

    a. said irradiator (102) is configured to irradiate said second light according to a frequency scheme including a first sequence of frequencies increasing continuously from a first frequency to a second, greater, frequency, and a second sequence of frequencies decreasing continuously from the second frequency to said first frequency;
    b. said irradiator (102) is configured to irradiate said first light according to a frequency scheme including a first sequence of frequencies increasing continuously from a first frequency to a second, greater, frequency, and a second sequence of frequencies decreasing continuously from the second frequency to said first frequency;
    c. said irradiator (102) is configured to irradiate said first and/or second light from said first frequency of 100 Hz to said second frequency of 3000 Hz;
    d. said irradiator (102) is configured to irradiate said first light during a first irradiation period, and to irradiate said second light during a second irradiation period; wherein said first and second irradiation periods at least partially overlap.

13. A non-therapeutic method relating to cosmetic or aestetic light treatments, comprising the steps of:

    a. providing a wearable finger clip or toe clip apparatus, comprising:

        i. a finger clip or a toe clip, and an irradiator (102) having at least one LED source (122) and at least one pulsed laser light source (121), wherein the irradiator (102) is mounted in the clip for irradiating an area of interest;
        ii. a controller (103) comprising a machine readable medium for controlling said at least one LED source (122) and said at least one pulsed laser light source (121); and
        iii. a power source;

b. positioning said irradiator (102);

c. operating said irradiator (102) by said controller (103) to activate said at least one LED source (122) and said at least one pulsed laser light source (121); and

d. activating said at least one LED source (122) and said at least one pulsed light source (121) according to a predetermined protocol (500) including a first sequence of frequencies increasing linearly and continuously from a first frequency to a second, greater, frequency , and a second sequence of frequencies decreasing linearly and continuously from the second frequency to said first frequency;

wherein said step of activating light sources comprises the step of activating said at least one pulsed laser light source (121) to irradiate a second light at a wavelength of between 600 nm to 950 nm, and activating said at least one LED source (122) to irradiate a first light at a wavelength of between 390 nm and 500 nm;

wherein beams of said first and second light are configured to overlap the area of interest.

14. The method of claim 13, wherein said step of activating said light sources comprises the step of activating said at least one LED source (122) to irradiate the first light at a wavelength of 470 nm.

15. The method of claim 13, wherein said step of activating said light sources comprises the step of activating said at least one pulsed laser light source (121) to irradiate the second light at a wavelength of 905 nm.

16. The method of claim 13, wherein at least one of the following holds true:

a. irradiating said second light according to a frequency scheme including a first sequence of frequencies increasing continuously from a first frequency to a second, greater, frequency, and a second sequence of frequencies decreasing continuously from the second frequency to the first frequency;

b. irradiating said first light according to a frequency scheme including a first sequence of frequencies increasing continuously from a first frequency to a second, greater, frequency, and a second sequence of frequencies decreasing continuously from the second frequency to the first frequency;

c. activating light sources from said first frequency comprising a frequency of 100 Hz to said second frequency of 3000 Hz;

d. irradiating said first light during a first irradiation period, and irradiating said second light during a second irradiation period; wherein said first and second irradiation periods at least partially overlap.

17. The method of claim 13, wherein said step of providing said wearable finger clip or toe apparatus (101) comprises the step of providing a toe or finger clip having a lower portion and an up portion opposed to one another and pivotally attaching each portion to a spring portion to keep the clip closed around a toe or finger, wherein said clip incorporates said at least one LED source and said at least one pulsed laser light source mounted in the clip in order to emit said first and second light towards a nail when said clip is in a closed configuration around the toe or finger.

**Patentansprüche**

1. Tragbare Fingerclip- oder Zehenclipvorrichtung (101) zur Behandlung von Nagelpilzinfektionen, umfassend:

a. einen Fingerclip oder Zehenclip und ein Bestrahlungsgerät (102), das mindestens eine LED-Quelle (122) und mindestens eine Quelle von gepulstem Laserlicht (121) aufweist; wobei das Bestrahlungsgerät (102), zur Bestrahlung eines Gebiets von Interesse, in dem Clip montiert ist;

b. ein Steuergerät (103), umfassend ein maschinenlesbares Medium zur Steuerung der mindestens einen LED-Quelle und der Quelle von gepulstem Laserlicht (122, 121); und

c. eine Stromquelle;

wobei das Steuergerät (103) mit vorgegebenen Protokollen (500) zum Anweisen der mindestens einen LED-Quelle (122), erstes Licht auf einer vorgegebenen Wellenlänge von zwischen 390 nm bis 500 nm zu emittieren, und zum Anweisen der mindestens einen Quelle von gepulstem Laserlicht (121), zweites Licht auf einer vorgegebenen Wellenlänge von zwischen 600 nm bis 950 nm zu emittieren, versehen ist;

wobei weiterhin die mindestens eine LED-Quelle (122) und die mindestens eine Quelle von gepulstem Laserlicht (121) so in einer geometrischen Anordnung angeordnet sind, dass die projizierten Strahlen der mindestens einen LED-Quelle (122) und der mindestens einen Quelle von gepulstem Laserlicht (121) so eingerichtet sind, dass sie das Gebiet von Interesse mindestens teilweise überlappen, wodurch sie für maximale Effizienz sorgen.

2. Vorrichtung (101) nach Anspruch 1, wobei mindestens eines von folgendem gilt:

a. die geometrische Anordnung weist eine Konfiguration auf, ausgewählt aus der Gruppe bestehend aus: Vieleck, Quadrat, Dreieck, Sechseck, Kugel, Halbkugel, Zylinder, Kreis, Ellipse, Rechtecke, T-Form, Schmetterlingsform oder gleich welches Vieleck, das als zweidimensionale Anordnung angeordnet ist; und
b. die geometrische Anordnung weist eine dreidimensionale (3D) oder zweidimensionale Anordnungskonfiguration auf.

3. Vorrichtung (101) nach Anspruch 1, wobei der Abstand zwischen der mindestens einen LED-Quelle und der mindestens einen Quelle von gepulstem Laserlicht (121, 122) 6,9 mm beträgt.

4. Vorrichtung (101) nach Anspruch 1, wobei die mindestens eine LED-Quelle (122) dazu eingerichtet ist, Licht auf einer Wellenlänge von 470 nm zu emittieren.

5. Vorrichtung (101) nach Anspruch 1, wobei die mindestens eine Quelle von gepulstem Laserlicht (122) dazu eingerichtet ist, Licht auf einer Wellenlänge von 905 nm zu emittieren.

6. Vorrichtung (101) nach Anspruch 1, wobei das Steuergerät (103) dazu eingerichtet ist, die mindestens eine Quelle von gepulstem Laserlicht (121) in einem Pulswiederholratenmodus zu betreiben, während die mindestens eine LED-Lichtquelle in einem quasikontinuierlichen Modus betrieben wird.

7. Vorrichtung (101) nach Anspruch 1, wobei die geometrische Anordnung von Lichtquellen eine erweiterte Lichtquelle produziert, die einen durchschnittlichen Akzeptanzwinkel von 35 mrad aufweist.

8. Vorrichtung (101) nach Anspruch 1, wobei der Zehen- oder Fingerclip einen unteren Teil und einen oberen Teil beinhaltet, die einander gegenüberstehen, wobei jeder Teil schwenkbar an einem Federteil befestigt ist, um den Clip um eine Zehe oder einen Finger herum geschlossen zu halten, wobei der Clip dazu eingerichtet ist, besagtes erstes und zweites Licht zu einem Nagel hin zu emittieren, wenn der Clip sich in einer geschlossenen Konfiguration um die Zehe oder den Finger befindet.

9. Vorrichtung (101) nach Anspruch 1, eine Trennvorrichtung umfassend, die dazu eingerichtet ist, das Bestrahlungsgerät und/oder andere Teile der Vorrichtung mindestens teilweise von dem Gebiet von Interesse zu trennen.

10. Vorrichtung (101) nach Anspruch 1, wobei die mindestens eine LED-Quelle (122) dazu eingerichtet ist, eine Strahldivergenz von 9x25 Grad vorzusehen.

11. Vorrichtung (101) nach Anspruch 1, wobei die mindestens eine Quelle von gepulstem Laserlicht (121) dazu eingerichtet ist, eine Strahldivergenz von 120 Grad vorzusehen.

12. Vorrichtung (101) nach Anspruch 1, wobei mindestens eines von folgendem gilt:

a. das Bestrahlungsgerät (102) ist dazu eingerichtet, das zweite Licht gemäß einem Frequenzschema auszustrahlen, das eine erste Abfolge von Frequenzen, die von einer ersten Frequenz zu einer zweiten, größeren Frequenz kontinuierlich zunimmt, und eine zweite Abfolge von Frequenzen, die von der zweiten Frequenz zu der ersten Frequenz kontinuierlich abnimmt, beinhaltet;
b. das Bestrahlungsgerät (102) ist dazu eingerichtet, das erste Licht gemäß einem Frequenzschema auszustrahlen, das eine erste Abfolge von Frequenzen, die von einer ersten Frequenz zu einer zweiten, größeren Frequenz kontinuierlich zunimmt, und eine zweite Abfolge von Frequenzen, die von der zweiten Frequenz zu der ersten Frequenz kontinuierlich abnimmt, beinhaltet;
c. das Bestrahlungsgerät (102) ist dazu eingerichtet, das erste und/oder zweite Licht von besagter erster Frequenz von 100 Hz bis zu besagter zweiter Frequenz von 3000 Hz auszustrahlen;
d. das Bestrahlungsgerät (102) ist dazu eingerichtet, das erste Licht während eines ersten Bestrahlungszeitraums auszustrahlen und das zweite Licht währen eines zweiten Bestrahlungszeitraums auszustrahlen; wobei der erste und der zweite Bestrahlungszeitraum einander mindestens teilweise überlappen.

13. Nichttherapeutisches Verfahren betreffend kosmetische oder ästhetische Lichtbehandlungen, umfassend die Schritte des:

a. Vorsehens einer tragbaren Fingerclip- oder Zehenclip-Vorrichtung, umfassend:

i. einen Fingerclip oder Zehenclip, und ein Bestrahlungsgerät (102), das mindestens eine LED-Quelle (122) und mindestens eine Quelle von gepulstem Laserlicht (121) aufweist; wobei das Bestrahlungsgerät (102), zur Bestrahlung eines Gebiets von Interesse, in dem Clip montiert ist;

ii. ein Steuergerät (103), umfassend ein maschinenlesbares Medium zur Steuerung der mindestens einen LED-Quelle (122) und der mindestens einen Quelle von gepulstem Laserlicht (121); und

iii. eine Stromquelle;

b. Positionierens des Bestrahlungsgeräts (102);

c. Bedienens des Bestrahlungsgeräts (102) durch das Steuergerät (103) zur Aktivierung der mindestens einen LED-Quelle (122) und der mindestens einen Quelle von gepulstem Laserlicht (121); und

d. Aktivierens der mindestens einen LED-Quelle (122) und der mindestens einen Quelle von gepulstem Laserlicht (121) gemäß einem vorgegebenen Protokoll (500), das eine erste Abfolge von Frequenzen, die von einer ersten Frequenz zu einer zweiten, größeren Frequenz linear und kontinuierlich zunimmt, und eine zweite Abfolge von Frequenzen, die von der zweiten Frequenz zu der ersten Frequenz linear und kontinuierlich abnimmt, beinhaltet;

wobei der Schritt des Aktivierens von Lichtquellen den Schritt des Aktivierens der mindestens einen Quelle von gepulstem Laserlicht (121) zum Ausstrahlen eines zweiten Lichts auf einer Wellenlänge von zwischen 600 nm bis 950 nm, und Aktivierens der mindestens einen LED-Quelle (122) zum Ausstrahlen eines ersten Lichts auf einer Wellenlänge von zwischen 390 nm und 500 nm umfasst;

wobei Strahlen des ersten und des zweiten Lichts so eingerichtet sind, dass sie das Gebiet von Interesse überlappen.

14. Verfahren nach Anspruch 13, wobei der Schritt des Aktivierens der Lichtquellen den Schritt des Aktivierens der mindestens einen LED-Quelle (122) zum Ausstrahlen des ersten Lichts auf einer Wellenlänge von 470 nm umfasst.

15. Verfahren nach Anspruch 13, wobei der Schritt des Aktivierens der Lichtquellen den Schritt des Aktivierens der mindestens einen Quelle von gepulstem Laserlicht (122) zum Ausstrahlen des zweiten Lichts auf einer Wellenlänge von 905 nm umfasst.

16. Verfahren nach Anspruch 13, wobei mindestens eines von folgendem gilt:

a. Ausstrahlen des zweiten Lichts gemäß einem Frequenzschema, das eine erste Abfolge von Frequenzen, die von einer ersten Frequenz zu einer zweiten, größeren Frequenz kontinuierlich zunimmt, und eine zweite Abfolge von Frequenzen, die von der zweiten Frequenz zu der ersten Frequenz kontinuierlich abnimmt, beinhaltet;

b. Ausstrahlen des ersten Lichts gemäß einem Frequenzschema, das eine erste Abfolge von Frequenzen, die von einer ersten Frequenz zu einer zweiten, größeren Frequenz kontinuierlich zunimmt, und eine zweite Abfolge von Frequenzen, die von der zweiten Frequenz zu der ersten Frequenz kontinuierlich abnimmt, beinhaltet;

c. Aktivieren von Lichtquellen von der ersten Frequenz, umfassend eine Frequenz von 100 Hz, bis zu der zweiten Frequenz von 3000 Hz;

d. Ausstrahlen des ersten Lichts während eines ersten Bestrahlungszeitraums, und Ausstrahlen des zweiten Lichts während eines zweiten Bestrahlungszeitraums; wobei der erste und der zweite Bestrahlungszeitraum einander mindestens teilweise überlappen.

17. Verfahren nach Anspruch 13, wobei der Schritt des Vorsehens der tragbaren Fingerclip- oder Zehen-Vorrichtung (101) den Schritt umfasst des Vorsehens eines Zehen- oder Fingerclips mit einem unteren Teil und einem oberen Teil, die einander gegenüberstehen, und schwenkbar Befestigens jedes Teils an einem Federteil, um den Clip um eine Zehe oder einen Finger herum geschlossen zu halten, wobei der Clip die mindestens eine LED-Quelle und die mindestens eine Quelle von gepulstem Laserlicht enthält, die in dem Clip montiert sind, um das erste und das zweite Licht zu einem Nagel hin zu emittieren, wenn der Clip sich in einer geschlossenen Konfiguration um die Zehe oder den Finger befindet.

**Revendications**

1. Appareil portable (101) sous la forme d'une pince destinée à un doigt ou d'une pince destinée à un orteil pour traiter des infections unguéales de type fongique, comprenant :

a. une pince destinée à un doigt ou une pince destinée à un orteil et un irradiateur (102) possédant au moins une source DEL (122) et au moins une source de lumière laser pulsée (121), ledit irradiateur (102) étant monté dans la pince pour irradier une zone d'intérêt ;

b. un contrôleur (103) comprenant un support lisible par machine pour commander ladite au moins une source DEL et ladite au moins une source de lumière laser pulsée (122, 121) ; et

c. une source d'alimentation ;

dans lequel ledit contrôleur (103) est muni de protocoles prédéterminés (500) pour donner instruction à ladite au moins une source DEL (122) d'émettre une première lumière à une longueur d'onde prédéterminée entre 390 nm et 500 nm, et pour donner instruction à ladite au moins une source de lumière laser pulsée (121) d'émettre une deuxième lumière à une longueur d'onde prédéterminée entre 600 nm et 950 nm ; dans lequel, en outre, ladite au moins une source DEL (122) et ladite au moins une source de lumière laser pulsée (121) sont arrangées en un réseau géométrique de manière telle que les faisceaux projetés de ladite au moins une source DEL (122) et de ladite au moins une source de lumière laser pulsée (121) sont configurés pour recouvrir au moins en partie ladite zone d'intérêt, pour ainsi obtenir un rendement maximal.

2. Appareil (101) selon la revendication 1, dans lequel au moins une des conditions indiquées ci-après est remplie :

a. ledit réseau géométrique possède une configuration qui est choisie parmi le groupe constitué par : un polygone, un carré, un triangle, un hexagone, une sphère, un hémisphère, un cylindre, un cercle, une ellipse, un rectangle, la forme d'un T, un noeud papillon ou n'importe quel polygone disposé sous la forme d'un réseau bidimensionnel ; et

b. ledit réseau géométrique possède une configuration de réseau en trois dimensions (3D) ou de réseau bidimensionnel.

3. Appareil (101) selon la revendication 1, dans lequel la distance entre ladite au moins une source DEL et ladite au moins une source de lumière laser pulsée (122, 121) s'élève à 6,9 mm.

4. Appareil (101) selon la revendication 1, dans lequel ladite au moins une source DEL (122) est configurée pour émettre une lumière à une longueur d'onde de 470 nm.

5. Appareil (101) selon la revendication 1, dans lequel ladite au moins une source de lumière laser pulsée (121) est configurée pour émettre une lumière à une longueur d'onde de 905 nm.

6. Appareil (101) selon la revendication 1, dans lequel ledit contrôleur (103) est configuré pour activer ladite au moins une source de lumière laser pulsée (121) dans un mode de taux de répétition des impulsions, tandis que ladite au moins une source de lumière DEL est entraînée dans un mode en quasi-continu.

7. Appareil (101) selon la revendication 1, dans lequel ledit réseau géométrique des sources de lumière produit une source de lumière étendue possédant un angle d'admission moyen de 35 mR.

8. Appareil (101) selon la revendication 1, dans lequel la pince destinée à un doigt ou à un orteil englobe une portion inférieure et une portion supérieure, opposées l'une à l'autre, chaque portion étant fixée en pivotement à une portion faisant ressort pour maintenir la pince à l'état fermé autour d'un orteil ou d'un doigt, la pince étant configurée pour émettre ladite première et ladite deuxième lumière en direction d'un ongle lorsque la pince se trouve dans sa configuration fermée autour de l'orteil ou du doigt.

9. Appareil (101) selon la revendication 1, comprenant un séparateur qui est configuré pour séparer au moins en partie ledit irradiateur et/ou les autres portions de l'appareil de ladite zone d'intérêt.

10. Appareil (101) selon la revendication 1, dans lequel ladite au moins une source DEL (122) est configurée pour procurer une divergence de faisceau de 9 x 25 degrés.

11. Appareil (101) selon la revendication 1, dans lequel ladite au moins une source de lumière laser pulsée (121) est configurée pour procurer une divergence de faisceau de 120 degrés.

12. Appareil (101) selon la revendication 1, dans lequel au moins une des conditions indiquées ci-après est remplie :

a. ledit irradiateur (102) est configuré pour irradier ladite deuxième lumière conformément à un schéma de fréquences englobant une première séquence de fréquences qui augmentent en continu depuis une première fréquence jusqu'à une deuxième fréquence supérieure et une deuxième séquence de fréquences qui diminuent en continu depuis la deuxième fréquence jusqu'à ladite première fréquence ;

b. ledit irradiateur (102) est configuré pour irradier ladite première lumière conformément à un schéma de fréquences englobant une première séquence de fréquences qui augmentent en continu depuis une première fréquence jusqu'à une deuxième fréquence supérieure et une deuxième séquence de fréquences qui diminuent en continu depuis la deuxième fréquence jusqu'à ladite première fréquence ;

c. ledit irradiateur (102) est configuré pour irradier ladite première lumière et/ou ladite deuxième lumière à partir de ladite première fréquence de 100 Hz jusqu'à ladite deuxième fréquence de 3000 Hz;

d. ledit irradiateur (102) est configuré pour irradier ladite première lumière au cours d'une première période d'irradiation et pour irradier ladite deuxième lumière au cours d'une deuxième période d'irradiation, lesdites première et deuxième périodes d'irradiation se chevauchant au moins en partie.

13. Procédé non thérapeutique concernant des traitements cosmétiques ou esthétiques faisant appel à de la lumière, comprenant les étapes dans lesquelles :

a. on procure un appareil portable sous la forme d'une pince destinée à un doigt ou d'une pince destinée à un orteil, comprenant :

i. une pince destinée à un doigt ou une pince destinée à un orteil et un irradiateur (102) possédant au moins une source DEL (122) et au moins une source de lumière laser pulsée (121), ledit irradiateur (102) étant monté dans la pince pour irradier une zone d'intérét ;

ii. un contrôleur (103) comprenant un support lisible par machine pour commander ladite au moins une source DEL (122) et ladite au moins une source de lumière laser pulsée (121) ; et

iii. une source d'alimentation ;

b. on positionne ledit irradiateur (102) ;

c. on actionne ledit irradiateur (102) via ledit contrôleur (103) pour activer ladite au moins une source DEL (122) et ladite au moins une source de lumière laser pulsée (121) ; et

d. on active ladite au moins une source DEL (122) et ladite au moins une source de lumière laser pulsée (121) conformément à un protocole prédéterminé (500) englobant une première séquence de fréquences qui augmentent de manière linéaire et de manière continue à partir d'une première fréquence jusqu'à une deuxième fréquence supérieure, et une deuxième séquence de fréquences qui diminuent de manière linéaire et de manière continue à partir de la deuxième fréquence jusqu'à ladite première fréquence ;

dans lequel ladite étape d'activation des sources de lumière comprend l'étape dans laquelle on active ladite au moins une source de lumière laser pulsée (121) pour irradier une deuxième lumière à une longueur d'onde entre 600 nm et 950 nm, et dans lequel on active ladite au moins une source DEL (122) pour irradier une première lumière à une longueur d'onde entre 390 nm et 500 nm ;

dans lequel les faisceaux de ladite première et de ladite deuxième lumière sont configurés pour recouvrir la zone d'intérêt.

14. Procédé selon la revendication 13, dans lequel ladite étape dans laquelle on active lesdites sources de lumière comprend l'étape dans laquelle on active ladite au moins une source DEL (122) pour irradier la première lumière à une longueur d'onde de 470 nm.

15. Procédé selon la revendication 13, dans lequel ladite étape dans laquelle on active lesdites sources de lumière comprend l'étape dans laquelle on active ladite au moins une source de lumière laser pulsée (121) pour irradier la deuxième lumière à une longueur d'onde de 905 nm.

16. Procédé selon la revendication 13, dans lequel au moins une des conditions indiquées ci-après est remplie :

a. l'irradiation de ladite deuxième lumière conformément à un schéma de fréquences englobant une première séquence de fréquences qui augmentent en continu depuis une première fréquence jusqu'à une deuxième fréquence supérieure et une deuxième séquence de fréquences qui diminuent en continu depuis la deuxième fréquence jusqu'à ladite première fréquence ;

b. l'irradiation de ladite première lumière conformément à un schéma de fréquences englobant une première

**EP 2 749 317 B1**

séquence de fréquences qui augmentent en continu depuis une première fréquence jusqu'à une deuxième fréquence supérieure et une deuxième séquence de fréquences qui diminuent en continu depuis la deuxième fréquence jusqu'à la première fréquence ;

c. l'activation des sources de lumière à partir de ladite première fréquence comprenant une fréquence de 100 Hz jusqu'à ladite deuxième fréquence de 3000 Hz ;

d. l'irradiation de ladite première lumière au cours d'une première période d'irradiation et l'irradiation de ladite deuxième lumière au cours d'une deuxième période d'irradiation, lesdites première et deuxième périodes d'irradiation se chevauchant au moins en partie.

17. Procédé selon la revendication 13, dans lequel ladite étape dans laquelle on procure ledit appareil portable (101) sous la forme d'une pince destinée à un doigt ou à un orteil comprend l'étape dans laquelle on procure une pince destinée à un doigt ou à un orteil possédant une portion inférieure et une portion supérieure, opposées l'une à l'autre, et dans laquelle on fixe en pivotement chaque portion à une portion faisant ressort pour maintenir la pince à l'état fermé autour d'un orteil ou d'un doigt, ladite pince possédant à l'état incorporé ladite au moins une source DEL et ladite au moins une source de lumière laser pulsée, montées dans la pince dans le but d'émettre ladite première et ladite deuxième lumière en direction d'un ongle, lorsque ladite pince se trouve dans sa configuration fermée autour de l'orteil ou du doigt.

**Figure1**

**Figure 2**

310

302

315

312

318

Fig. 3A

302

320

301

312

315

Fig. 3B

# Figure3

Figure 4A

Figure 4B

Figure 4C

**Figure 5**

**Figure 6**

Providing a portable apparatus for treating fungal nail infection

710

Positioning said irradiator to a subject infected nail,

720

Operating the irradiator to irradiate light of at least first light source and second light source on at least part of said nail

730

Irradiating said light sources according to a predetermined protocol

740

**Figure 7**

804

801

12 mm          12 mm

803          12 mm          802

**Figure 8A**

7 mm

810          830          820

70
mm

**Figure 8B**

9D          9C          9B          9A

Figure 9

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2007167999 A
- US 2007104664 A
- US 2008058905 A

- US 2008076958 A
- US 2009234270 A
- WO 2013005156 A1